(19) Europäisches Patentamt
European Patent Office
Office européen des brevets

(11) EP 0 474 211 B1

(12) **EUROPÄISCHE PATENTSCHRIFT**

(45) Veröffentlichungstag und Bekanntmachung des Hinweises auf die Patenterteilung:
**17.01.1996 Patentblatt 1996/03**

(51) Int Cl.$^6$: **C12P 35/00**, C12P 35/06

(21) Anmeldenummer: **91114933.4**

(22) Anmeldetag: **04.09.1991**

(54) **Verfahren zur kontinuierlichen Umsetzung von Cephalosporinderivaten zu Glutaryl-7-amino-cephalosporansäurederivaten**

Process for the continuous transformation of cephalosporin derivatives in glutaryl-7-amino-cephalosporin derivatives

Procédé pour la transformation en continu de dérivés de céphalosporine en dérivés de glutaryl-7-amino-céphalosporine

(84) Benannte Vertragsstaaten:
**AT BE CH DE DK ES FR GB GR IT LI LU NL SE**

(30) Priorität: **05.09.1990 DE 4028119**

(43) Veröffentlichungstag der Anmeldung:
**11.03.1992 Patentblatt 1992/11**

(73) Patentinhaber:
**HOECHST AKTIENGESELLSCHAFT**
**D-65926 Frankfurt am Main (DE)**

(72) Erfinder:
- **Bayer, Thomas, Dr.**
  **D-6232 Bad Soden am Taunus (DE)**
- **Sauber, Klaus, Dr.**
  **D-6232 Bad Soden am Taunus (DE)**

(56) Entgegenhaltungen:
**WO-A-90/12110** **DE-A- 1 939 341**
**FR-A- 2 133 927**

- **CHEMICAL ABSTRACTS, Band 105, Nr. 3, 21. Juli 1986, Columbus, OH (US); M. SOMOROVA, Seite 536, Abstrakt 23098p/**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 474 211 B1

Printed by Jouve, 75001 PARIS (FR)

## Beschreibung

Cephalosporin C (3-Acetoxymethyl-7β-(D-5-amino-5-carboxypentanamido)-ceph-3-em-4-carbonsäure) kann mit permeabilisierten Zellen der Hefe Trigonopsis variabilis zu α-Ketoadipinyl-7-aminocephalosporansäure oxidiert werden und anschließend mit Wasserstoffperoxid zu Glutaryl-7-aminocephalosporansäure oxidativ decarboxyliert werden (DOS 2 219 454). Unter den dort beschriebenen Bedingungen werden Reaktionszeiten von 0,5 bis 3 Stunden benötigt und eine Ausbeute von 60 bis 73 % erreicht.

D-Aminosäure-Oxidase E.C. 1.4.3.3 (im folgenden DAO genannt) katalysiert die oxidative Desaminierung von D-Aminosäuren zu den entsprechenden α-Ketosäuren, Ammoniak und Wasserstoffperoxid.

Neben der kommerziell erhältlichen DAO aus Schweinenieren wird das Enzym von Bakterien, Hefen und Pilzen synthetisiert. Unter diesen zeichnet sich Trigonopsis variabilis als guter DAO-Produzent aus. Neben der Verwendung dieses Enzyms zur Racemat-Trennung von D,L-Aminosäuren und dem quantitativen Nachweis von D-Aminosäuren in verschiedenen Lösungen ist die Fähigkeit zur oxidativen Desaminierung von Cephalosporin C besonders hervorzuheben.

Nach der belgischen Patentschrift 736 934 wird DAO aus Pilzen gewonnen und muß zur erwähnten Oxidation von Cephalosporin C durch Lyse freigesetzt werden.

In der deutschen Offenlegungsschrift 22 19 454 (US-Patentschrift 3 801 458) wird die Herstellung von Cephalosporin C-Derivaten mit Hilfe von aktivierten Zellen von Trigonopsis variabilis CBS 4095 beschrieben. "Aktiviert" bedeutet hier, daß die Hefezellen einem physikalischen und/oder chemischen Verfahren unterzogen wurden, so daß die in den Zellen enthaltene DAO zur Katalyse der Oxidation von Cephalosporin C (CPC) zugänglich wird, jedoch nicht substantiell freigesetzt wird.

Aufgabe der vorliegenden Erfindung war es ein Verfahren zur weitgehend vollständigen Umsetzung von Cephalosporin-Derivaten zu den entsprechenden Glutaryl-7-aminocephalosporansäure-Derivaten zu finden, bei dem der bekannte Zerfall von Cephalosporin in wäßrigen Lösungen weitgehend verhindert, die Stabilität des verwendeten DAO-haltigen Katalysators verbessert und eine hohe Ausbeute an Glutaryl-7-Aminocephalosporansäure (G-7-ACS) erreicht wird.

Es wurde nun ein Verfahren gefunden, mit dem Cephalosporin-Derivate zu Glutaryl-7-aminocephalosporansäurederivaten umgesetzt werden können, das dadurch gekennzeichnet ist, daß die Reaktion durch DAO-haltigen Katalysator kontinuierlich durchgeführt wird und gegebenenfalls anschließend Wasserstoffperoxid zugegeben wird.

Überraschenderweise konnte durch die kontinuierliche Verfahrensweise eine wesentliche Verbesserung der Katalysatorausnutzung im Vergleich zum "Satz"-Verfahren erreicht werden. Ferner erhöhte sich die Standzeit des DAO-haltigen Katalysators bei der kontinuierlichen Zugabe der Substrate deutlich im Vergleich zum Satz-Verfahren. Auch die Verweilzeit von CPC in den Reaktionsgefäßen konnte verringert werden.

Die Erfindung betrifft somit ein Verfahren zur Umsetzung von Cephalosporin-Derivaten zu den entsprechenden Glutaryl-7-Aminocephalosporansäurederivaten, das dadurch gekennzeichnet ist, daß die Reaktion kontinuierlich in Gegenwart von DAO-haltigem Katalysator durchgeführt wird und gegebenenfalls anschließend Wasserstoffperoxid zugegeben wird.

In den erfindungsgemäßen Verfahren können alle Cephalosporin-Derivate eingesetzt werden, solange sie eine D-5-amino-5-carboxypentanamido-Gruppe an Position 7 des Cephalosporinringes besitzen. Bevorzugt werden Cephalosporinderivate der Formel II,

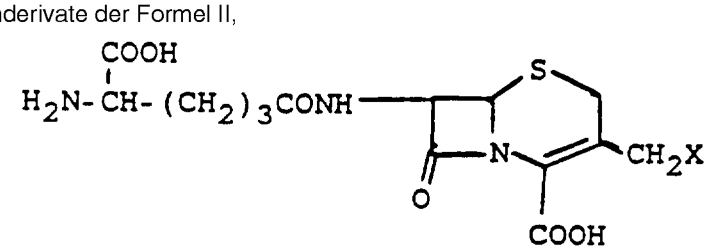

(II)

in welcher

X für eine Acetatgruppe,
den Rest eines Nukleophils,
einen Heterozyklus,
eine Hydroxygruppe oder
Wasserstoff steht,

sowie Salze der Cephalosporinderivate der Formel II zu den Verbindungen der Formel I,

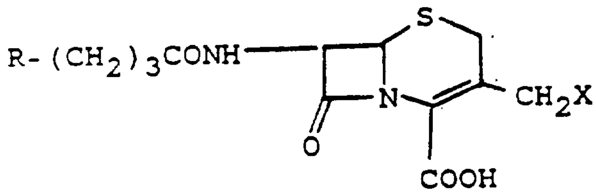

(I)

in welcher

X die obengenannte Bedeutung hat,
R für eine Carboxygruppe oder
eine Ketocarboxygruppe steht,
sowie Salze der Cephalosporinderivate der Formel I, umgesetzt.

Die Verbindungen der Formel II können sowohl in verhältnismäßig unreinem Zustand, als auch in vorgereinigter Form, eingesetzt werden.

Unter dem Begriff Nucleophil sind beispielsweise Verbindungen wie Pyridin oder tertiäre Amine gemeint. Mit dem Begriff Heterozyklus sind Verbindungen wie z.B. Thiazolylderivate, Pyrimidine, 5,6-Dihydrocylopentan[b]pyridin, Thienyl, Pyridyl, Pyridazinyl, Thiazolinyl, Pyrazinyl, Indolinyl oder Indasolyl gemeint. Salze der Verbindungen der Formel I oder II sind z.B. Zink, Ammoniumsalze oder Salze der Alkali- oder Erdalkalimetalle, wie Natrium, Kalium, Calcium oder Magnesium. Unter dem Begriff "DAO-haltiger Katalysator" werden immobilisierte D-Aminosäure-Oxidasen, rohes Enzym, permeabilisierte oder aktivierte DAO-haltige Zellen, grob aufgeschlossen, DAO-haltige Zellen oder isolierte, gereinigte DAO verstanden. Die Aktivierung oder Permeabilisierung erfolgt wie in US 3,801,458 beschrieben oder nach bekannten physikalischen oder chemischen Methoden.

D-Aminosäure-Oxidase (DAO) kann aus zahlreichen Organismen gewonnen werden, beispielsweise aus Mikroorganismen, Pflanzen, Pilzen oder tierischen Organen, wie Schweineniere. In dem erfindungsgemäßen Verfahren hat sich die DAO aus Trigonopsis als besonders geeignet herausgestellt. Insbesondere DAO aus Trigonopsis variabilis CBS 4095 kann im Verfahren eingesetzt werden.

Die Herstellung der DAO aus Trigonopsis variabilis erfolgt durch Fermentation wie z.B. in der US Patentschrift 3,801,458 beschrieben. Die Anzucht der Zellen erfolgt in einem komplexen Nährmedium, das Glucose, Hefeextrakt, Kaliumphosphat, übliche Salze und Spurenelemente enthält, sowie Methionin oder Alanin als Stickstoffquelle.

Die DAO kann als gereinigtes Enzym, isolierter Rohextrakt, Zellextrakt, in Form von grob aufgeschlossenen Zellen oder mit anderen Enzymen zusammen immobilisiert werden. Die Trigonopsis variabilis Zellen können beispielsweise nach der Kultivierung durch chemische oder physikalische Methoden aufgebrochen werden (U.S. Patent 3,801,458) und dann nach bekannten Verfahren immobilisiert werden. Beispielsweise kann eine Immobilisierung durch Einschluß in Polysaccharide wie z.B. Alginat, Agar, Chitosan oder Carrageenan erfolgen oder durch Einschluß in Polymere wie Acryl-Polymer z.B. Polyacrylamide oder vernetzte Polyamine. Das vernetzte Polyamin ist vorzugsweise ein mit einem Di- oder Polyaldehyd wie Glutaraldehyd vernetztes inertes Protein wie z.B. Albumin oder Gelatine. Es können ebenso Polysaccharide wie Chitin oder Chitosan, die mit einem Di- oder Polyaldehyd bzw. Polyphosphat vernetzt sind, eingesetzt werden.

Für die Immobilisierung von gereinigten, teilweise gereinigten oder rohen Zellextrakten, die DAO enthalten kommen z.B. trägergebundene Immobilisierungsverfahren in Frage. Beispielsweise kann die DAO durch eine covalente Bindung über einen zur Katalyse nicht essentiellen Lysinrest an den polymeren Träger gekuppelt werden. Eine weitere Möglichkeit ist, die Adsorption der DAO an einen Träger sowie die anschließende Quervernetzung mit z.B. Glutardialdehyd.

Als Enzymträger kommen polymere, poröse Träger wie Cellulosen, z.B. DEAE- oder CM-Cellulosen, Sepharosen, wie z.B. BrCN- oder Divinylsulfonaktivierte Sepharosen, modifizierte Polyacrylamidgele mit Amino- oder Hydroxylgruppen oder verschiedene organische Copolymerisate aus Acrylamid, Methacrylaten oder Methacrylamid und Maleinsäureanhydrid in Frage. Ferner können als Enzymträger auch Copolymere aus Glycidylmethacrylat, Allylglycidylether, Methylenbismethylacrylamid und Methacrylamid, wie z.B. ®Eupergit, eingesetzt werden.

Bevorzugte Enzymträger sind vernetzte Polymere auf Basis von Polyvinylester und Polyvinylalkoholen nach der DOS 33 44 912. Die Verankerungsreaktion zwischen DAO und Enzymträger erfolgt in bekannter Weise, wie etwa in DE 2 215 687 beschrieben. Zumeist erfolgt die Umsetzung bei Raumtemperatur bzw. bei +40°C oder darunter liegenden Temperaturen, insbesondere bei Temperaturen unterhalb von +10°C, vorzugsweise bei 0 bis +5°C.

Die Verankerungsreaktion erfolgt vorzugsweise in der Umgebung eines neutralen pH-Wertes, beispielsweise bei pH-Werten von 5 bis 9. In der Regel ist es auch nicht erforderlich, stärker saure oder alkalische Bedingungen einzuhalten, da die makroporösen Perlpolymerisate auch bereits im Neutralbereich mit der DAO schnell reagieren. Die dabei entstehende Bindung bietet genügend Stabilität für lange Lagerungen und hohe Operationsstabilität.

Beim erfindungsgemäßen Verfahren geht man am besten so vor, daß man den DAO-haltigen Katalysator in ein, zwei oder mehrere Reaktionsgefäße füllt, die miteinander verbunden sind und kontinuierlich die Substrate durch das oder die Reaktionsgefäße leitet. Beispielsweise wird die Reaktionslösung über eine Rührkesselkaskade geleitet. Die Gefäße bestehen aus einem inerten Material das mit den Substraten und Produkten nicht reagiert. Die Gefäße sind z.B. Rührkessel, Blasensäulen oder Schlaufenreaktoren und können mit einem Kühl/Heizmantel umgeben sein. Die Gefäße besitzen Ein- und Auslaßöffnungen für den Substrat- und Produktstrom und sind so angeordnet, daß es im Reaktionsgefäß zu einer guten Durchmischung kommen kann. Die Gefäße können über eine zusätzliche Öffnung mit Sauerstoff oder einer sauerstoffhaltigen Gasmischung, z.B. Luft, $O_2$ angereicherter Luft oder Stickstoff/Sauerstoff-Mischungen versorgt werden. Gegebenenfalls kann die Reaktionslösung mit Hilfe eines Rührers gemischt werden. Die Reaktionsgefäße werden mit einer entsprechenden Rückhalteeinrichtung wie z.B. Fritte, Membran oder Filtersieb versehen, dessen Durchlässigkeit so gewählt wird, daß ein Abfließen des DAO-haltigen Katalysators weitgehend verhindert wird. Die Reaktionsgefäße sind auch mit entsprechenden Öffnungen versehen, so daß eine Entsorgung des nicht verbrauchten Gases möglich ist. Die Reaktionsgefäße besitzen ferner Meßstellen zur Verfolgung des Reaktionsverlaufes, z.B. Messung des pH-Wertes oder des Sauerstoffpartialdruckes.

Die Substratlösung wird in das erste Reaktionsgefäß gepumpt. Hier erfolgt die von der DAO katalysierte Reaktion von Cephalosporinderivaten mit Sauerstoff zum entsprechenden α-Ketoadipinyl-7-amino-cephalosporansäurederivat, Ammoniak und Wasserstoffperoxid. Die entstandenen Produkte und noch nicht umgesetzte Cephalosporinderivate können in das nächste Reaktionsgefäß gepumpt werden. Diese Verfahrensweise wird wiederholt bis die Cephalosporinderivate weitgehend umgesetzt sind. Ferner findet in den Reaktionsgefäßen mit dem entstandenen Wasserstoffperoxid und α-Ketoadipinyl-7-aminocephalosporansäurederivat (KA-7-ACS) eine oxidative Decarboxylierung zu Glutaryl-7-aminocephalosporansäure statt. Falls es nicht zu einer vollständigen Umsetzung von KA-7-ACS kommt kann in einem nachgeschalteten Reaktionsgefäß Wasserstoffperoxid kontrolliert zudosiert werden, so daß die Reaktion weitgehend vollständig zu G-7-ACS abläuft. Durch die Zugabe von Wasserstoffperoxid wird das Redoxpotential im Reaktionsgefäß verändert. Im kontinuierlichen Betrieb kann über eine Redoxelektrode der zur vollständigen Umsetzung erforderliche Wasserstoffperoxidgehalt gemessen und über eine entsprechende Kontrolleinrichtung durch Nachdosieren von Wasserstoffperoxid konstant gehalten werden. Durch diese Verfahrensweise wird eine Überdosierung vermieden, die sonst zu Nebenreaktionen, wie z.B. Oxidation von Schwefel, führen kann.

Die Zugabe der Cephalosporinderivate zum Reaktionsansatz kann in fester Form oder als Lösung in Wasser mit gegebenenfalls zusätzlichen Pufferkomponenten erfolgen. Die Konzentration der Cephalosporinderivate kann in weiten Grenzen schwanken, beispielsweise zwischen 0,001 und 1 M, bevorzugt zwischen 0,01 und 0,1 M.

Die eingeführte Sauerstoffmenge kann zwischen 1 und 500 l $O_2$ pro Stunde und l Reaktionslösungsvolumen, bevorzugt 10 bis 100 l $O_2$ pro Stunde und l Reaktionslösungsvolumen. betragen. Die eingesetzte DAO Konzentration liegt zwischen 10 und 5000 Units (U) pro Liter Reaktionsgefäßvolumen. Der DAO-haltige Katalysator wird zwischen 0,1 und 95 Gew.-%, bevorzugt 0,5 bis 50 %, insbesondere 1 bis 20 %, eingesetzt.

Es wird vorteilhaft bei einem pH-Wert zwischen 5 und 9, bevorzugt zwischen 6,0 und 8,5, gearbeitet. Es ist außerdem zweckmäßig die Reaktion in einem Temperaturbereich von 4 bis 65°C, insbesondere 10 bis 50°C, durchzuführen. Die günstigste Vorgehensweise hängt von dem jeweiligen verwendeten DAO-haltigen Katalysator ab und kann in einfachen Vorversuchen leicht festgelegt werden.

Die Verweilzeit der Reaktionslösung in einem Gefäß kann zwischen 5 min und 800 min, bevorzugt 20 bis 120 min, liegen. Das Verfahren kann unter sterilen oder nicht sterilen Bedingungen durchgeführt werden.

Die Reaktionsprodukte können nach Reinigung oder auch im ungereinigten Zustand durch bekannte chemische oder enzymatische Umsetzung in 7-Aminocephalosporansäure (7-ACS) überführt werden. 7-ACS ist Ausgangssubstrat für eine Vielzahl von semisynthetischen Antibiotika.

Im folgenden wird die Erfindung anhand von Beispielen näher erläutert. Prozentangaben beziehen sich auf das Gewicht.

Beispiel 1

Als Vorkultur wird Trigonopsis variabilis CBS 4095 in folgender Nährlösung kultiviert:

| | |
|---|---|
| Glucose | 20 g/l (separat autoklaviert) |
| D,L-Methionin | 4 g/l (separat autoklaviert) |
| $KH_2PO_4$ | 2 g/l |
| $K_2HPO_4$ | 0,2 g/l |

Fortsetzung der Tablelle auf der nächsten Seite

(fortgesetzt)

| | |
|---|---|
| $MgSO_4$ x $7H_2O$ | 0,5 g/l |
| $CaCl_2$ x $6H_2O$ | 0,1 g/l |
| NaCl | 0,1 g/l |
| Spurenelementlösung | 10 ml |
| Vitaminlösung | 1 ml (nach autoklavieren zugeben) |
| pH 6,0 | |

Vitaminlösung:

| | |
|---|---|
| Biotin | 20 mg/l |
| Thiamin | 100 mg/l |
| in ethanol (50 %ig) gelöst | |

Spurenelementlösung:

| | |
|---|---|
| Borsäure | 5 g/l |
| $MnCl_2$ x 4 $H_2O$ | 2 g/l |
| $CuCl_2$ x $3H_2O$ | 2 g/l |
| $ZnSO_4$ x $7H_2O$ | 1 g/l |
| $FeCl_3$ x $6H_2O$ | 3,4 g/l |
| in Aqua bidest. gelöst | |

Als 1%iges Inokulum dient eine NaCl-Abschwemmung vom Schrägröhrchen mit einer $OD_{578nm}$ = 18. Die Vorkulturdauer beträgt 24 Stunden bei 30°C und 190 Upm.

Die Fermentation wird unter folgenden Bedingungen durchgeführt:

Nährlösung:

Die Nährlösung entspricht dem der Vorkultur, jedoch auf folgenden Mengen aufgestockt:

| | |
|---|---|
| Glucose | 30 g/l |
| D,L-Methionin | 6 g/l |
| ®Desmophen | 0,1 % (bei Bedarf) |

Fermentationsbedingungen:

| | |
|---|---|
| Inokulum | 2,5 - 5 % |
| Temperatur | 28°C |
| Fermentationszeit | 50 - 60 h |

Zur DAO-Bestimmung werden 0,4 g Zellen eingefroren, gefolgt von Auftauen bei saurem pH, z.B. etwa pH 3-4; das Frieren kann bei einer Temperatur unter -10°C, z.B. etwa -20°C durchgeführt werden. Es sollte genügend lange gefroren werden, um die Freisetzung der DAO aus den Zellen hervorzurufen, z.B. zumindest 1 Stunde bei -20°C.

Die Aktivität wird mit folgendem Testansatz photometrisch bestimmt:

<u>Lösungen</u>:

| | |
|---|---|
| 1) Puffer | 100 mM KPP; pH 7,3; luftgesättigt |
| 2) o-Phenylendiamin | 0.02 % in $H_2O$ |
| 3) Peroxidase | 1 mg/ml in Puffer |
| 4) Enzym | Optimal: 0,5 - 1,0 Units/ml |
| 5) Substrat | 150 mM Na - CPC (100 %) in Puffer |

Testdurchführung:

$\lambda$ = 405 nm (Maximum)
$\varepsilon$ = 4020 l/mol*cm
$\nu$ = 30°C

| Volumen: | Endkonzentration: |
|---|---|
| 1) 2.00 ml | 83 mM |
| 2) 0.50 ml | 0.0034 % |
| 3) 0.10 ml | 0.034 mg/ml |
| 4) 0.05 ml | |
| 2 min warten | |
| 5) 0.30 ml | 15.25 mM |
| 2.95 ml | |

Berechnung:

$$\frac{\text{Units}}{\text{ml}} = \frac{\Delta E * \text{Verdünnung} * \text{Gesamtvolumen}}{\text{min} * \varepsilon * d * \text{Probenvolumen}}$$

Unter den obengenannten Bedingungen wird eine Enzymaktivität im Fermenter von 200 U/l erreicht.

Beispiel 2

Zur Durchführung einer heterogen-vernetzenden Perlcopolymerisation wurde eine Lösung von 80 g Vinylacetat, 20 g Divinylethylenharnstoff, 1 g Azoisobutyronitril und 200 g n-Heptanol in der Lösung von 0,175 g $NaH_2PO_4$, 3 g $Na_2HPO_4$ und 5 g Polyvinylpyrrolidon in 500 ml Wasser dispergiert und polymerisiert. Nach vier Stunden wurde das Verdünnungsmittel durch Wasserdampfdestillation entfernt und das Produkt isoliert. Die Ausbeute betrug 77,7 g an völlig rundem klarem Perlpolymerisat. Der mittlere Partikeldurchmesser lag bei etwa 30 µm (Rührgeschwindigkeit von 460 upm).

Das Produkt hatte ein Schüttvolumen von 1,55 ml/g. Das verseifte Produkt hatte ein Schüttelvolumen von 1,54 ml/g, es quoll in Wasser auf 5 ml/g.

20 g des hydrolysierten Perlcopolymerisates ließ man in 200 ml Epichlorhydrin 24 Stunden bei Raumtemperatur quellen. Anschließend wurde unter langsamen Rühren die Temperatur auf 113 bis 115°C erhöht und 4 Stunden gehalten.

Nach Abkühlen wurde über eine Nutsche filtriert und das Copolymerisat mehrmals jeweils 1 Stunde in Aceton ausgerührt. Das acetonfeuchte Copolymerisat wurde bis zur Gewichtskonstanz im Vakuumschrank bei 50°C getrocknet. Das Epoxidäquivalent betrug 244 (gemessen nach Axen: Acta Chem. Scand. B 29 (1975) Nr. 4).

Beispiel 3

Zu 100 mg eines nach Beispiel 2 hergestellten Trägers wurden 500 µl einer DAO-haltigen Lösung (20 U/ml) zugesetzt. Zur Einstellung der Enzymlösung auf pH 7,8 wurde 1-molarer Kaliumphosphatpuffer zugegeben. Die Dauer der Fixierung des Enzyms an den Träger betrug 72 Stunden bei 25°C. Anschließend wurde die nicht kovalent an den Träger gebundene DAO über eine Glasfritte abgesaugt und der Rückstand mehrmals mit 1-molarer Natriumchloridlösung, dann mit Pufferlösung ausgewaschen. Die Ausbeute an nutschenfeuchtem Material lag bei 324 mg. Die Aktivität wurde wie

in Beispiel 1 angegeben gemessen und ergab einen Wert von 18 U/g im Feuchtzustand.

Beispiel 4

Die Zellen von Trigonopsis variabilis CBS 4095 werden nach den in Beispiel 1 angegebenen Bedingungen gezüchtet und durch Einfrieren und Auftauen permeabilisiert. Anschließend wird 1 g Zellfeuchtmasse mit 10 ml einer 1%igen wäßrigen Chitosanlösung vermischt und immobilisiert, indem die Mischung tropfenweise in eine 2%ige wäßrige Natriumtripolyphosphatlösung (pH 8,0; 25°C) gegeben wird. Man erhält eine DAO-Aktivität von 2,5 U/g Katalysator-Feuchtmasse.

Beispiel 5

A Enzymatische Umsetzung in einem Reaktionsgefäß DAO wird wie in Beispiel 3 beschrieben immobilisiert und in einem 1,5 l Glasgüfäß mit Rührer und Doppelmantel mit Cephalosporin C inkubiert. Es wurden folgende Reaktionsparameter eingehalten:

| | |
|---|---|
| Arbeitsvolumen | 1 l |
| Temperatur | 25 °C |
| DAO-Konzentration | 4 % Immobilisat (Beispiel 3) |
| | 720 U/l |
| Sauerstoff | 50 l/h |
| pH-Wert | 7,0 |
| Cephalosporin C | 30 mM |
| Rührerdrehzahl | 300 Upm |
| Reaktionszeit | 110 min |

Nach 110 min waren 85 % des eingesetzten Cephalosporin C umgesetzt worden.

Die Reaktionslösung wurde von der immobilisierten DAO abgetrennt und das Enzym wurde mit frischer Substratlösung versehen. Nach 32 Stunden war der Umsatz auf weniger als 50 % gefallen.

B Enzymatische Umsetzung in zwei hintereinander geschalteten Reaktionsgefäßen

DAO wird wie in Beispiel 3 beschrieben immobilisiert und in zwei 1 l Glasgefäßen mit Doppelmantel und Rührer gefüllt. Die Cephalosporin-Lösung wurde kontinuierlich in das erste Reaktionsgefäß gepumpt und von dort ins zweite Reaktionsgefäß geleitet. Es wurden folgende Reaktionsparameter eingehalten:

| | |
|---|---|
| Arbeitsvolumen/Gefäß | 0,5 l |
| Temperatur | 25 °C |
| DAO-Konzentration | 4 % Immobilisat (Beispiel 3) |
| | 720 U/l |
| Sauerstoff | 30 l/h |
| pH-Wert | 7,0 |
| Cephalosporin C | 30 mM |
| Rührerdrehzahl | 300 Upm |
| Verweilzeit/Gefäß | 50 min |
| Gesamt-Verweilzeit | 100 min |

Nach 100 min waren 85 % des eingesetzten Cephalosporin C umgesetzt worden. Ein Austausch des Katalysators war erst nach 48 Stunden nötig, weil dann weniger als 85 % des eingesetzten Cephalosporins umgesetzt wurden.

Tabelle 1 zeigt den Vergleich von Verfahren A zu dem erfindungsgemäßen Verfahren B.

Tabelle 1

| | Verfahren A | erfindungsgemäßes Verfahren B |
|---|---|---|
| Ausbeute | 1,0 | 1,0 |
| Katalysatorausnutzung | 1,0 | 1,5 |
| Reaktionszeit | 1,0 | 0;9 |
| Raum-Zeit-Ausbeute | 1,0 | 1,1 |

**Patentansprüche**

1. Verfahren zur Umsetzung von Cephalosporin-Derivaten mit einer D-5-amino-5-carboxypentanamidogruppe an Position 7 des Cephalosporinringes zu den entsprechenden Glutaryl-7-aminocephalosporansäurederivaten, dadurch gekennzeichnet, daß die Reaktion kontinuierlich in Gegenwart von D-Aminosäure-Oxidase (E.C.I.4.3.3.) -haltigem Katalysator durchgeführt wird und gegebenenfalls anschließend Wasserstoffperoxid zugegeben wird.

2. Verfahren nach Anspruch 1, dadurch gekennzeichnet, daß die Reaktionslösung über eine Rührkesselkaskade geleitet wird.

3. Verfahren nach Anspruch 1 oder 2, dadurch gekennzeichnet, daß D-Aminosäure-Oxidase aus Pilzen verwendet wird.

4. Verfahren nach einem oder mehreren der Ansprüche 1 bis 3, dadurch gekennzeichnet, daß Cephalosporinderivate der Formel II

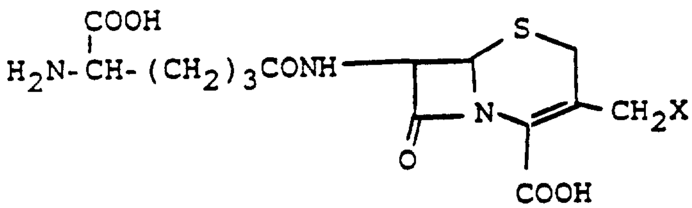

(II)

X für eine Acetatgruppe,
der Rest eines Nukleophils,
einen Heterozyklus,
eine Hydroxygruppe oder
Wasserstoff steht, sowie Salze der
Cephalosporinderivate der Formel II,

zu den Verbindungen der Formel I

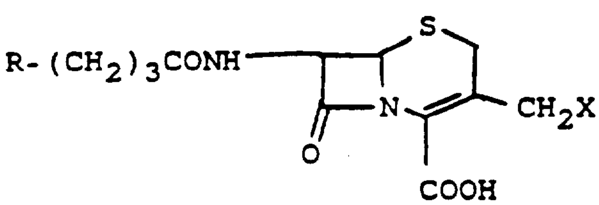

(I)

in welcher X die obengenannte Bedeutung hat,

R für eine Carboxygruppe oder
Ketocarboxygruppe steht, sowie Salze der Cephalosporinderivate der Formel I,

umgesetzt wird.

5. Verfahren nach einem oder mehreren der Ansprüche 1 bis 4, dadurch gekennzeichnet, daß die D-Aminosäure-Oxidase mit Polysacchariden immobilisiert wird.

6. Verfahren nach Anspruch 5, dadurch gekennzeichnet, daß Alginat, Carrageenan oder Chitosan verwendet wird.

7. Verfahren nach einem oder mehreren der Ansprüche 1 bis 6, dadurch gekennzeichnet, daß ein Enzymträger aus einem Copolymerisat aus Polyvinylester und Polyvinylalkohol oder aus Glycidylmethacrylat, Allylglycidylether, Methacrylamid und Methylenbismethacrylamid verwendet wird.

8. Verfahren nach einem oder mehreren der Ansprüche 1 bis 7, dadurch gekennzeichnet, daß aktivierte oder permeabilisierte D-Aminosäure-Oxidase enthaltene Zellen eingesetzt werden.

9. Verfahren nach einem oder mehreren der Ansprüche 1 bis 8, dadurch gekennzeichnet, daß die Wasserstoffperoxidzugabe kontinuierlich erfolgt.

**Claims**

1. A process for converting cephalosporin derivatives having a D-5-amino-5-carboxypentanamido group on position 7 of the cephalosporin ring into the corresponding glutaryl-7-aminocephalosporanic acid derivatives, which comprises carrying out the reaction continuously in the presence of D-amino-acid oxidase (E.C.1.4.3.3.)-containing catalyst and, where appropriate, subsequently adding hydrogen peroxide.

2. The process as claimed in claim 1, wherein the reaction solution is passed through a cascade of stirred vessels.

3. The process as claimed in claim 1 or 2, wherein D-amino-acid oxidase from fungi is used.

4. The process as claimed in one or more of claims 1 to 3, wherein cephalosporin derivatives of the formula II

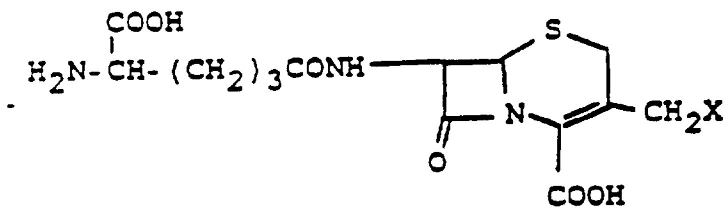

(II)

in which X is an acetate group,
the radical of a nucleophile,
a heterocycle,
a hydroxyl group or
hydrogen, and salts of the cephalosporin derivatives of the formula II, are converted into the compounds of the formula I

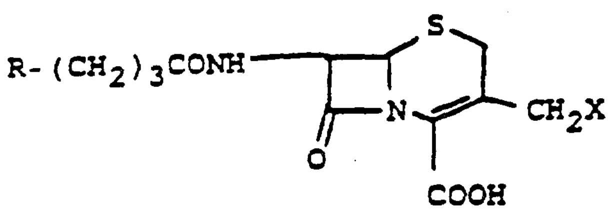

(I)

in which X has the abovementioned meaning, R is a carboxyl group or a keto carboxyl group, and salts of the cephalosporin derivatives of the formula I.

5. The process as claimed in one or more of claims 1 to 4, wherein the D-amino-acid oxidase is immobilized with polysaccharides.

6. The process as claimed in claim 5, wherein alginate, carrageenan or chitosan is used.

7. The process as claimed in one or more of claims 1 to 6, wherein an enzyme carrier composed of a copolymer of polyvinyl ester and polyvinyl alcohol or of glycidyl methacrylate, allyl glycidyl ether, methacrylamide and methylenebismethacrylamide is used.

8. The process as claimed in one or more of claims 1 to 7, wherein activated or permeabilized cells containing D-amino-acid oxidase are employed.

9. The process as claimed in one or more of claims 1 to 8, wherein the hydrogen peroxide is added continuously.

**Revendications**

1. Procédé de conversion de dérivés de type céphalosporine avec un groupe D-5-amino-5-carboxypentanamido en position 7 du noyau céphalosporine en dérivés correspondants de type glutarylamino-7-céphalosporine, caractérisé en ce que la réaction à réaliser en continu en présence d'un catalyseur contenant de la D-aminoacide-oxydase (E.C.1.4.3.3.) et que l'on ajoute ensuite éventuellement du péroxyde d'hydrogène.

2. Procédé selon la revendication 1, caractérisé en ce que le milieu réactionnel est amené sur la cascade d'un récipient d'agitation.

3. Procédé selon la revendication 1 ou 2, caractérisé en ce que l'on utilise de la D-aminoacide-oxydase issue de champignons.

4. Procédé selon l'une ou plusieurs des revendications 1 à 3, caractérisé en ce que les dérivés de céphalosporine de formule II

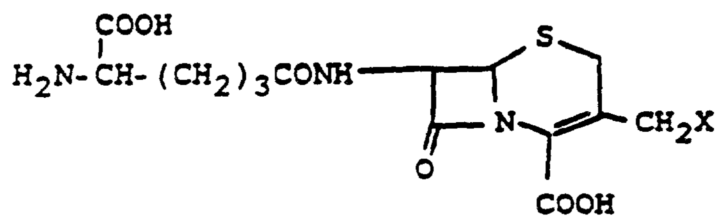

(II)

dans laquelle X représente un groupe acétate, le résidu d'un nucléophile, un hétérocycle, un groupe hydroxy ou l'hydrogène, ainsi que des sels des dérivés de céphalosporine de formule II, sont convertis en composés de formule I

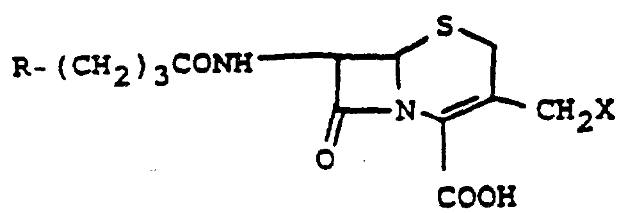

(I)

dans laquelle X a les significations mentionnées ci-dessus, et R représente un groupe carboxy ou cétocarboxy ainsi que des sels des dérivés de céphalosporine de formule I.

5. Procédé selon l'une ou plusieurs des revendications 1 à 4, caractérisé en ce que la D-aminoacide-oxydase est immobilisée sur des polysaccharides.

6. Procédé selon la revendication 5, caractérisé en ce que l'on utilise un alginate, un carraghénane ou un chitosane.

7. Procédé selon l'une ou plusieurs des revendications 1 à 6, caractérisé en ce que l'on utilise un support pour enzyme obtenu à partir d'un copolymère de poly(ester vinylique) et de poly(alcool vinylique) ou obtenu à partir de méthacrylate de glycidyle, d'éther d'allyle et de glycidyle, de méthacrylamide et de méthylènebisméthacrylamide.

8. Procédé selon l'une ou plusieurs des revendications 1 à 7, caractérisé en ce que l'on utilise des cellules activées ou perméabilisées contenant de la D-aminoacide-oxydase.

**9.** Procédé selon l'une ou plusieurs des revendications 1 à 8, caractérisé en ce que l'on procède de façon continue à l'addition de peroxyde d'hydrogène.